# EUROPÄISCHE PATENTSCHRIFT

(11) **EP 1 440 664 B1**
(45) Veröffentlichungstag und Bekanntmachung des Hinweises auf die Patenterteilung: **09.05.2007**
(21) Anmeldenummer: 03029512.5
(22) Anmeldetag: 20.12.2003
(51) Int. Cl.: A61B 17/72, A61B 17/80

(54) **Implantat für Osteosynthese**
Implant for osteosynthesis
Implant pour l'ostéosynthèse

(30) Priorität: 23.01.2003 DE 20300987 U
(43) Veröffentlichungstag der Anmeldung: 28.07.2004
(73) Patentinhaber: Stryker Trauma GmbH, 24232 Schönkirchen (DE)
(72) Erfinder: Prager, Ronald, Dipl.-Ing., 24796 Bovenau (DE); Zander, Nils, Dipl.-Ing., 24340 Eckernförde (DE)
(74) Vertreter: Kopf, Korbinian Paul

(56) Entgegenhaltungen:
- DE-A- 3 244 243
- DE-U- 9 321 544
- DE-U- 20 307 265
- US-A- 3 744 488

## Beschreibung

Die Erfindung bezieht sich auf ein Implantat für die Osteosynthese nach dem Oberbegriff des Anspruchs 1, wie es beispielsweise aus der DE-A-3244243 als bekannt hervorgeht.

Es ist allgemein bekannt, Platten zur Versorgung von Knochenfrakturen mit Hilfe von Knochenschrauben an dem Knochen zu fixieren. Es ist ferner seit langem bekannt, Knochennägel zur Versorgung von Frakturen der Röhrenknochen als so genannte Verriegelungsnägel auszuführen. Verriegelungsnägel haben sowohl am distalen als auch am proximalen Ende Querbohrungen zur Aufnahme einer Knochen- oder Verriegelungsschraube. Es ist bereits bekannt geworden, die Bohrungen zur Aufnahme einer Knochenschraube mit einem Gewinde zu versehen. Das Gewinde ist zumeist ein so genanntes Kortikalisgewinde, d.h. ein dem Gewinde der Knochenschraube entsprechendes Gewinde. Es hat den Vorteil, dass es das postoperative Auswandern der Knochenschrauben hemmt.

Es ist bekannt, Nägel zur Versorgung von Frakturen des Humerus als Verriegelungsnägel auszuführen und im proximalen Teil des Verriegelungsnagels mehrere Querbohrungen vorzusehen, die in Umfangsrichtung versetzt zueinander liegen und ggf. schräg zur Achse des Nagels verlaufen. Bei einer Humerusfraktur ist das Auswandern von Verriegelungsschrauben kritisch und somit ein besonderes Problem.

Der Erfindung liegt die Aufgabe zugrunde, ein Implantat für die Osteosynthese zu schaffen, bei dem das postoperative Auswandern von Knochenschrauben wirksam verhindert wird.

Diese Aufgabe wird durch die Merkmale des Anspruchs 1 gelöst.

Bei dem erfindungsgemäßen Implantat weist die Gewindebohrung eine Ringnut auf, deren Durchmesser größer ist als der Gewindeaußendurchmesser und die einen Ring aus verformbarem Material aufnimmt mit einem Innendurchmesser, der kleiner ist als der Außendurchmesser des Gewindes der Knochenschraube.

Der Ring für das Implantat nach der Erfindung ist vorzugsweise aus Kunststoffmaterial, zum Beispiel Polyethylen. Er versperrt einen Teil des Gewindequerschnitts und wird beim Eindrehen der Knochenschraube so verformt, dass ein Reibschluss zwischen der Knochenschraube einerseits und dem Ring und der Ringnut andererseits erzeugt wird, der die Knochenschraube an einem selbständigen Herausdrehen hindert.

Darüber hinaus wird die Knochenschraube durch den Ring in der Gewindebohrung zentriert, sodass gegenüber der nicht gesicherten Ausführung eine erhöhte Winkelstabilität der Knochenschraube im Implantat erreicht wird.

Üblicherweise haben die Bohrungen im Implantat ein Einlauf- und ein Auslaufende. Nach einer Ausgestaltung der Erfindung ist die Ringnut nahe dem Auslaufende angeordnet. Der Bohrungsabschnitt zwischen Auslaufende und der Ringnut ist nach einer weiteren Ausgestaltung der Erfindung vorzugsweise gewindefrei. Auch der Abschnitt nahe dem Einlaufende ist nach einer Ausgestaltung der Erfindung gewindefrei. Die Anordnung des Ringes auf der Ausgangsseite der Durchgangsbohrung stellt sicher, dass die Schraube sich schon im Gewindeeingriff befindet, wenn sie auf den Ring auftrifft.

Hierbei drückt die zumeist konische Spitze der Knochenschraube den Ring in die Nut hinein, bevor der Ring vom Gewinde der Knochenschraube verformt wird. Dadurch ist sichergestellt, dass der Ring nicht von der Verriegelungsschraube aus der Bohrung herausgedrückt wird.

Nach einer anderen Ausgestaltung der Erfindung ist die Ringnut im Querschnitt rechteckförmig, wobei der Ring einen komplementären Querschnitt aufweist. Allerdings ist vorzuziehen, dass die Breite des Ringes etwas geringer ist als die Breite der Ringnut. Dadurch lässt sich der Ring leichter in die Ringnut einsetzen. Außerdem ist ein gewisser Freiraum für die axiale Verformung des Ringes geschaffen.

Nach einer weiteren Ausgestaltung der Erfindung ist der Ring geteilt, und die Enden des Ringes haben einen Abstand voneinander, wenn der Ring im entspannten Zustand ist. Beim Einsetzen kann der Ring etwas radial kontrahiert werden, damit er ohne Probleme in die Ringnut einsetzbar ist. Damit der Ring nicht aus der Nut herausgedrückt wird, weisen beide Innenkanten eine Fase auf. Im Grunde wäre nur die dem Einlaufende zugekehrte Kante mit einer Fase zu versehen, da es jedoch gleich ist, in welcher Orientierung der Ring in die Nut eingesetzt ist, werden vorzugsweise beide Kanten mit einer Fase geformt.

Der Außendurchmesser des Ringes ist im entspannten Zustand etwas größer als der Durchmesser der Ringnut. Dadurch wird der Ring von selbst in der Nut gehalten, und zwar auch während des Transports und bei der Handhabung des Implantats vor dem Eindrehen der Knochenschraube.

Die Erfindung ist auf beliebige Implantate, die in Verbindung mit Knochenschrauben eingesetzt werden, verwendbar. Besonders bevorzugt ist die Erfindung bei einem intramedullären Implantat, etwa einem Knochennagel, vorzugsweise einem Verriegelungsnagel. Besonders vorteilhaft ist die Anwendung auf einen Humerusnagel, dessen proximales Ende drei oder mehr Querbohrungen aufweist, deren Achsen in Umfangsrichtung zueinander versetzt sind.

Die Erfindung soll anhand eines in Zeichnungen dargestellten Ausführungsbeipiels näher erläutert werden.
Fig. 1 zeigt perspektivisch das aufgeschnittene proximale Ende eines Humerusnagels mit Sicherungsringen nach der Erfindung.
Fig. 2 zeigt einen Längsschnitt durch einen Humerusnagel mit einer Gewindebohrung und einem Sicherungsring nach der Erfindung.
Fig. 3 zeigt einen Schnitt durch den Ring nach Fig. 4 entlang der Linie 3-3.
Fig. 4 zeigt die Vorderansicht eines Sicherungsrings nach Fig. 1 oder 2.

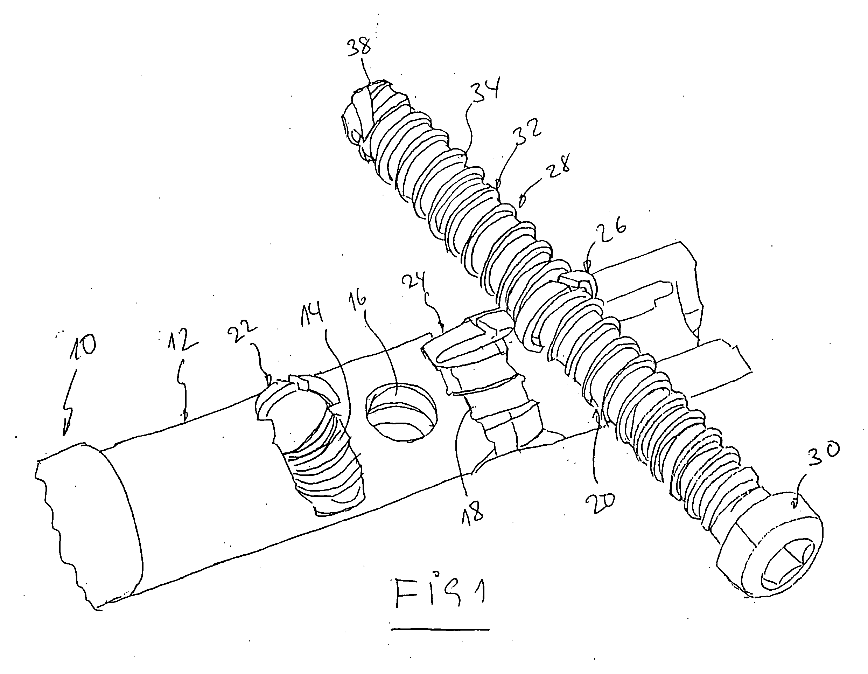

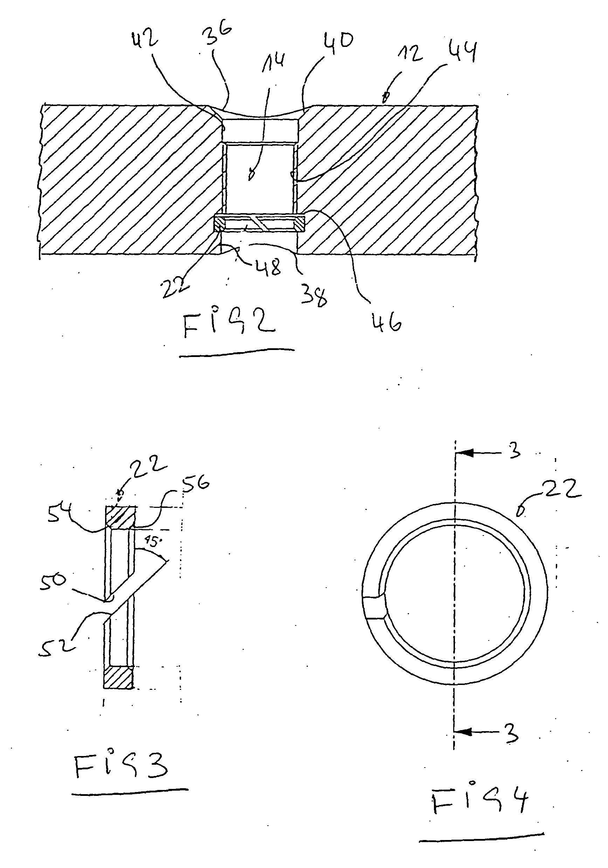

In Fig. 1 ist ein Humerusnagel 10 angedeutet mit einem längs aufgeschnittenen proximalen Abschnitt 12. Wie erkennbar, ist der Abschnitt 12 mit vier Querbohrungen 14 bis 20 versehen, die in Achsrichtung und Umfangsrichtung versetzt zueinander liegen und einen Winkel zur Längsachse des Nagels 10 aufweisen. Die Bohrungen 14 bis 20 sind als Gewindebohrungen ausgeführt. In Fig. 1 ist ferner zu erkennen, dass die Bohrungen 14, 18 und 20 jeweils einen Sicherungsring 22, 24 bzw. 26 aufnehmen. Sie dienen zur Sicherung einer Verriegelungsschraube 28, die einen Kopf 30 und einen Schaft 32 aufweist mit einem Gewinde 34. Das Gewinde 34 ist ein Kortikalisgewinde, das geeignet ist, einen festen Sitz im Knochen zu gewährleisten, ohne den Knochen unnötig zu belasten. Das Gewinde 34 ist ein Flachgewinde und wirkt mit dem Gewinde in den Gewindebohrungen 14 bis 20 zusammen.

In Fig. 2 ist eine einzige Gewindebohrung dargestellt, beispielsweise die Gewindebohrung 14. Die Bohrung 14 weist ein Einlaufende 36 und ein Auslaufende 38 auf. Die in Fig. 1 gezeigte Verriegelungsschraube 28 wird daher mit ihrer konischen Spitze 38 in das Einlaufende 36 eingeführt, das, wie bei 40 zu erkennen, einen Einlaufkonus 40 hat. An den Einlaufkonus 40 schließt sich ein gewindefreier Abschnitt 42 an. Danach folgt ein Gewindeabschnitt 44. An dessen Ende ist eine im Querschnitt rechteckige Ringnut 46 eingeformt, in welcher der Sicherungsring 22 aufgenommen ist. Zum Auslaufende 38 hin schließt sich wiederum ein gewindefreier Abschnitt 48 an. Wie zu erkennen, ist die Breite des Ringes 22 etwas geringer als die Breite der Ringnut 46.

In den Figuren 3 und 4 ist der Ring 22 etwas deutlicher herausgestellt. Wie zu erkennen, ist der Ring 22 geteilt, wobei die in einem Winkel von 45° mit einer Schräge versehenen Enden 50, 52 einen gewissen Abstand voneinander haben. Die Innenkanten des Ringes 22 sind mit einer Fase 54 bzw. 56 versehen. Der Außendurchmesser des Ringes 22 ist im entspannten Zustand geringfügig größer als der Durchmesser der Ringnut 46. Der Innendurchmesser ist kleiner als der Außendurchmesser des Gewindeabschnitts 34, jedoch größer als der Innendurchmesser des Gewindeabschnitts 34 der Schraube 28.

Beim Einsetzen wird der Ring radial etwas zusammengedrückt, sodass er leicht in die Ringnut 46 eingebracht werden kann. Anschließend entspannt er sich ein wenig, jedoch nicht bis zu seinem Durchmesser im entspannten Zustand, wodurch er einigermaßen sicher in der Ringnut 46 gehalten ist.

Wird eine Verriegelungsschraube 28 vom Einlaufende 36 in die Bohrung 14 eingeführt, kommen Gewinde 44 und 34 miteinander in Eingriff, bevor das Gewinde 34 mit dem Sicherungsring 22 zusammenwirkt. Die konische Spitze 38 der Verriegelungsschraube 28 drückt den Sicherungsring 22 zunächst radial nach außen, bevor das Gewinde 34 mit dem Ring 22 zusammenwirkt, sodass vermieden wird, dass der Ring 22 aus der Bohrung herausgedrückt wird. Der aus elastischem Kunststoffmaterial bestehende Ring wird entsprechend verformt, und es wird ein Reibschluss erzeugt zwischen dem Gewinde 34 und dem Ring 22 bzw. zwischen dem Ring 22 und der Ringnut 46, sodass eine wirksame Sicherung der Schraube 28 in der Bohrung 34 erzielt wird.

## Patentansprüche

1. Implantat für die Osteosynthese mit einem Implantatkörper, der mindestens eine Bohrung mit Gewinde aufweist und einer Knochenschraube, die mit dem Gewinde zusammenwirkt, wenn sie zur Befestigung des Implantatkörpers in einen Knochen eingeschraubt wird, **dadurch gekennzeichnet, dass** die Gewindebohrung (14 bis 20) eine Ringnut (46) aufweist, deren Durchmesser größer ist als der Gewindeaußendurchmesser und die einen Ring (22, 24, 26) aus verformbarem Material aufnimmt mit einem Innendurchmesser, der kleiner ist als der Außendurchmesser des Gewindes (34) der Knochenschraube (28).

2. Implantat nach Anspruch 1, **dadurch gekennzeichnet, dass** die Gewindebohrung (14) ein Einlauf- und ein Auslaufende (36, 38) aufweist und die Ringnut (46) nahe dem Auslaufende (38) angeordnet ist.

3. Implantat nach Anspruch 2, **dadurch gekennzeichnet, dass** der Bohrungsabschnitt (48) zwischen der Ringnut (46) und dem Auslaufende (38) gewindefrei ist.

4. Implantat nach einem der Ansprüche 1 bis 3, **dadurch gekennzeichnet, dass** der Bohrungsabschnitt (42) nahe dem Einlaufende (36) gewindefrei ist.

5. Implantat nach Anspruch 3 und 4, **dadurch gekennzeichnet, dass** die gewindefreien Abschnitte (42, 48) einen Durchmesser aufweisen, der geringfügig größer ist als der Außendurchmesser des Bohrungsgewindes (44).

6. Implantat nach einem der Ansprüche 1 bis 5, **dadurch gekennzeichnet, dass** die Ringnut (46) im Querschnitt rechteckig ist.

7. Implantat nach einem der Ansprüche 1 bis 6, **dadurch gekennzeichnet, dass** der Ring (22, 24, 26) aus einem elastisch nachgebendem Kunststoff geformt ist.

8. Implantat nach einem der Ansprüche 1 bis 7, **dadurch gekennzeichnet, dass** der Ring (22, 24, 26) geteilt ist und die Enden des Rings einen Abstand voneinander haben, wenn er im entspannten Zustand ist.

9. Implantat nach Anspruch 8, **dadurch gekennzeichnet, dass** die Enden (50, 52) des Rings (22) schräg sind und den gleichen Schrägungswinkel aufweisen.

10. Implantat nach einem der Ansprüche 1 bis 9, **dadurch gekennzeichnet, dass** die inneren Kanten des Rings (22) eine Fase (54, 56) aufweisen.

11. Implantat nach einem der Ansprüche 1 bis 10, **dadurch gekennzeichnet, dass** die Breite des Rings (22) etwas kleiner ist als die Breite der Ringnut (46).

12. Implantat nach einem der Ansprüche 8 bis 11, **dadurch gekennzeichnet, dass** der Außendurchmesser des Rings (22) im entspannten Zustand etwas größer ist als der Durchmesser der Ringnut (46).

13. Implantat nach einem der Ansprüche 1 bis 12, **dadurch gekennzeichnet, dass** der Implantatkörper ein Knochennagel ist.

14. Implantat nach Anspruch 13, **dadurch gekennzeichnet, dass** der Knochennagel ein Verriegelungsnagel (10) ist.

15. Implantat nach Anspruch 13 oder 14, **dadurch gekennzeichnet, dass** der Nagel ein Humerusnagel (10) ist, dessen proximales Ende (12) Querbohrungen (14, 16, 28) aufweist, deren Achsen in Umfangsrichtung zueinander versetzt sind.

## Claims

1. Implant for osteosynthesis with an implant body which has at least one bore with a thread and a bone screw which interacts with the thread when it is threaded into a bone for the fixation of the implant body, **characterised in that** the threaded bore (14 to 20) has an annular groove (46) the diameter of which is larger than the outer diameter of the thread and which accommodates a ring (22, 24, 26) made of deformable material with an inner diameter which is smaller than the outer diameter of the thread (34) of the bone screw (28).

2. Implant according to claim 1, **characterised in that** the threaded bore (14) comprises an inlet end and an outlet end (36, 38) and the annular groove (46) is disposed close to the outlet end (38).

3. Implant according to claim 2, **characterised in that** the bore portion (48) between the annular groove (46) and the outlet end (38) is non-threaded.

4. Implant according to any one of claims 1 to 3, **characterised in that** the bore portion (42) close to the inlet end (36) is non-threaded.

5. Implant according to claims 3 and 4, **characterised in that** the non-threaded portions (42, 48) have a diameter which is slightly larger than the outer diameter of the bore thread (44).

6. Implant according to any one of claims 1 to 5, **characterised in that** the annular groove (46) is rectangular in cross section.

7. Implant according to any one of claims 1 to 6, **characterised in that** the ring (22, 24, 26) is formed from a resiliently yielding plastic.

8. Implant according to any one of claims 1 to 7, **characterised in that** the ring (22, 24, 26) is split and the ends of the ring are spaced from each other when it is in relaxed state.

9. Implant according to claim 8, **characterised in that** the ends (50, 52) of the ring (22) are oblique and have the same angle of inclination.

10. Implant according to any one of claims 1 to 9, **characterised in that** the inner edges of the ring (22) have a chamfer (54, 56).

11. Implant according to any one of claims 1 to 10, **characterised in that** the width of the ring (22) is slightly smaller than the width of the of the annular groove (46).

12. Implant according to any one of claims 8 to 11, **characterised in that** the outer diameter of the ring (22) in relaxed state is slightly larger than the diameter of the annular groove (46).

13. Implant according to any one of claims 1 to 12, **characterised in that** the implant body is a bone nail.

14. Implant according to claim 13, **characterised in that** the bone nail is an interlocking nail (10).

15. Implant according to claim 13 or 14, **characterised in that** the nail is a humeral nail (10) the proximal end (12) of which has cross holes (14, 16, 28) the axes of which are offset from each other in the circumferential direction.

## Revendications

1. Implant pour l'ostéosynthèse, comprenant un corps muni d'au moins un trou taraudé, et une vis ostéochirurgicale qui coopère avec le taraudage lorsqu'elle est vissée dans un os en vue de la fixation du corps de l'implant, **caractérisé par le fait que** le trou taraudé (14 à 20) comporte une saignée annulaire (46) dont le diamètre est plus grand que le diamètre extérieur du taraudage et qui reçoit une bague (22, 24, 26) en un matériau déformable, offrant un diamètre intérieur plus petit que le diamètre extérieur du filetage (34) de la vis ostéochirurgicale (28).

2. Implant selon la revendication 1, **caractérisé par le fait que** le trou taraudé (14) comprend des extrémités respectives de commencement et d'achèvement (36, 38), et la saignée annulaire (46) se trouve à proximité de l'extrémité d'achèvement (38).

3. Implant selon la revendication 2, **caractérisé par le fait que** le segment (48) du trou, interposé entre la saignée annulaire (46) et l'extrémité d'achèvement (38), est dépourvu de taraudage.

4. Implant selon l'une des revendications 1 à 3, **caractérisé par le fait que** le segment (42) du trou, proche de l'extrémité de commencement (36), est dépourvu de taraudage.

5. Implant selon les revendications 3 et 4, **caractérisé par le fait que** les segments (42, 48) dépourvus de taraudage présentent un diamètre légèrement plus grand que le diamètre extérieur du taraudage (44) du trou.

6. Implant selon l'une des revendications 1 à 5, **caractérisé par le fait que** la saignée annulaire (46) est de section transversale rectangulaire.

7. Implant selon l'une des revendications 1 à 6, **caractérisé par le fait que** la bague (22, 24, 26) est venue de moulage en une matière plastique douée de souplesse élastique.

8. Implant selon l'une des revendications 1 à 7, **caractérisé par le fait que** la bague (22, 24, 26) est scindée, et les extrémités de ladite bague sont distantes l'une de l'autre à l'état soulagé de cette dernière.

9. Implant selon la revendication 8, **caractérisé par le fait que** les extrémités (50, 52) de la bague (22) sont inclinées et présentent le même angle d'obliquité.

10. Implant selon l'une des revendications 1 à 9, **caractérisé par le fait que** les arêtes intérieures de la bague (22) possèdent un chanfrein (54, 56).

11. Implant selon l'une des revendications 1 à 10, **caractérisé par le fait que** la largeur de la bague (22) est sensiblement plus petite que la largeur de la saignée annulaire (46).

12. Implant selon l'une des revendications 8 à 11, **caractérisé par le fait que** le diamètre extérieur de la bague (22) est sensiblement plus grand, à l'état soulagé, que le diamètre de la saignée annulaire (46).

13. Implant selon l'une des revendications 1 à 12, **caractérisé par le fait que** le corps dudit implant est une broche ostéochirurgicale.

14. Implant selon la revendication 13, **caractérisé par le fait que** la broche ostéochirurgicale est une broche de verrouillage (10).

15. Implant selon la revendication 13 ou 14, **caractérisé par le fait que** la broche est une broche humérale (10) dont l'extrémité proximale (12) comporte des trous transversaux (14, 16, 28) dont les axes sont mutuellement décalés dans le sens périphérique.
